# FASCICULE DE BREVET EUROPEEN

(11) **EP 2 934 319 B1**
(45) Date de publication et mention de la délivrance du brevet: **29.05.2019**
(21) Numéro de dépôt: 13815512.2
(22) Date de dépôt: 20.12.2013
(51) Int. Cl.: A61B 6/00

(54) **EMBASE POUR CASSETTE RADIOLOGIQUE NUMERIQUE PORTABLE**
SITZ FÜR EINE TRAGBARE DIGITALE RADIOLOGISCHE KASSETTE
SEATING FOR A PORTABLE DIGITAL RADIOLOGICAL CASSETTE

(30) Priorité: 21.12.2012 FR 1262665
(43) Date de publication de la demande: 28.10.2015
(73) Titulaire: Trixell, 38430 Moirans (FR)
(72) Inventeur: RIEUVERNET, Pierre, 38330 Montbonnot Saint Martin (FR)
(74) Mandataire: Collet, Alain
(86) Numéro de dépôt international: PCT/EP2013/077858
(87) Numéro de publication internationale: WO 2014/096430

(56) Documents cités:
- WO-A1-95/10411
- US-A1- 2007 272 873
- US-A1- 2011 204 239
- US-A1- 2012 168 632

## Description

L'invention concerne une cassette radiologique portable destinée à équiper un système radiologique numérique. La casette comprend un détecteur numérique de rayonnement ionisant permettant de fournir une image fonction du rayonnement reçu.

Le système radiologique comprend en outre une source de rayonnement ionisant, comme par exemple un tube à rayons X, permettant de générer un rayonnement X et une station de base comprenant un système de traitement de l'information permettant de synchroniser le tube à rayon X et le détecteur et permettant aussi de réaliser des traitements d'images comme de présenter à l'opérateur l'image corrigée de tous les défauts inhérents au détecteur et améliorée, par exemple par des traitements de rehaussement de contour. Un objet dont on veut obtenir l'image X est placé entre la source et le détecteur. Un tel système peut être utilisé dans de nombreuses applications telles que par exemple la radiologie médicale et le contrôle non destructif. L'invention peut également être mise en oeuvre pour d'autres types de rayonnements à détecter notamment des rayonnements gamma.

Par le passé, les systèmes radiologiques étaient volumineux et peu mobiles. Il était nécessaire de positionner l'objet par rapport au système pour obtenir l'image désirée. Avec l'apparition de détecteurs à l'état solide tels que par exemple décrit dans la demande de brevet français FR 2 605 166, le détecteur est devenu moins volumineux et il a été possible de déplacer le détecteur par rapport à un objet restant fixe. Pour la radiologie médicale, on a réalisé des détecteurs numériques sous forme de cassettes mobiles qu'il devient possible de placer à proximité immédiate d'un patient dont on veut réaliser une image, lorsque l'état de santé du patient empêche son déplacement vers une salle réservée à la radiologie.

La cassette mobile comprend essentiellement un détecteur numérique de rayonnement ionisant ayant la forme d'un panneau plat et une carte électronique assurant notamment le pilotage du détecteur numérique. Le détecteur et la carte sont disposés dans un boitier assurant leur protection mécanique.

La cassette utilisée dans un système portable subit beaucoup plus de manipulations que dans un système radiologique fixe et il est nécessaire de renforcer sa protection mécanique, notamment vis-à-vis de chocs que la cassette peut être amenée à subir lors de ses déplacements. Plus précisément, le détecteur numérique est souvent réalisé à partir de composant photosensibles disposés en matrice sur une dalle de verre formant l'élément le plus fragile de la cassette. Outre les chocs qui pourraient l'endommager, cette dalle est également sensible aux déformations notamment en torsion.

Les contraintes mécaniques auxquelles doivent résister la cassette et notamment le détecteur numérique obligent à renforcer la structure du boitier, ce qui se fait obligatoirement au détriment du poids de la cassette.

De plus, la mise en oeuvre d'une cassette radiologique présente des contraintes particulières en matière thermique. On s'est rendu compte que le fonctionnement du détecteur était altéré par la température ambiante.

Il est possible de corriger cette altération, par exemple en mesurant la température ambiante et en corrigeant globalement l'image issue du détecteur. Or la présence de la carte électronique dans le boitier de la casette à proximité immédiate du détecteur peut entrainer des artéfacts locaux dans l'image radiologique. Tout d'abord la carte électronique ne recouvre pas toute la surface du détecteur. La zone du détecteur faisant face à la carte électronique est donc plus affectée thermiquement que le reste du détecteur. Ensuite, localement dans la carte électronique, des écarts de température peuvent exister du fait de la présence de composants divers dont la dissipation thermique peut varier dans de grandes proportions. Il devient alors difficile de corriger les effets de ces écarts de température. Le document US2007/0272873 est considéré comme art antérieur le plus proche.

L'invention vise à améliorer la tenue mécanique et thermique des cassettes existantes tout en limitant leur augmentation de poids. L'invention est définie par la revendication 1.

A cet effet, l'invention a pour objet une cassette radiologique portable comprenant un détecteur numérique de rayonnement ionisant sous forme d'un panneau plat, une carte électronique assurant la gestion du détecteur numérique et un boitier assurant la protection mécanique du détecteur et de la carte électronique, caractérisée en ce qu'elle comprend en outre une embase monobloc plane supportant le détecteur sur une première de ses faces principales et la carte électronique sur une seconde de ses faces principales, les deux faces principales étant opposées, et en ce que l'embase est formée d'un empilement hétérogène réalisé de façon à ce que la conductivité thermique surfacique de l'embase soit supérieure à la conductivité thermique transversale de l'embase, en ce que l'embase comprend deux peaux externes formant les deux faces principales et un matériau interne disposé entre les deux peaux externes, le matériau interne et les deux peaux externes ayant une surface sensiblement égale à celle du détecteur, et en ce que la conductivité thermique des peaux externes est supérieure à la conductivité thermique du matériau interne.

On entend par conductivité thermique surfacique la conductivité thermique de l'embase dans des directions portées par les deux faces principales de l'embase et on entend par conductivité thermique transversale la conductivité thermique de l'embase dans une direction perpendiculaire aux directions portées par les deux faces principales de l'embase.

L'invention sera mieux comprise et d'autres avantages apparaîtront à la lecture de la description détaillée d'un mode de réalisation donné à titre d'exemple, description illustrée par le dessin joint dans lequel :
la figure 1 représente un exemple de système radiologique mettant en oeuvre l'invention ;
la figure 2 représente en vue éclatée une première variante de cassette portable ;
la figure 3 représente en vue éclatée une seconde variante de cassette portable ;
la figure 4 représente plus en détail des éléments disposés à l'intérieur d'un boitier de la cassette ;
les figures 5 et 6 représentent une variante d'une embase formant le support des éléments disposés à l'intérieur du boitier.

Par souci de clarté, les mêmes éléments porteront les mêmes repères dans les différentes figures.

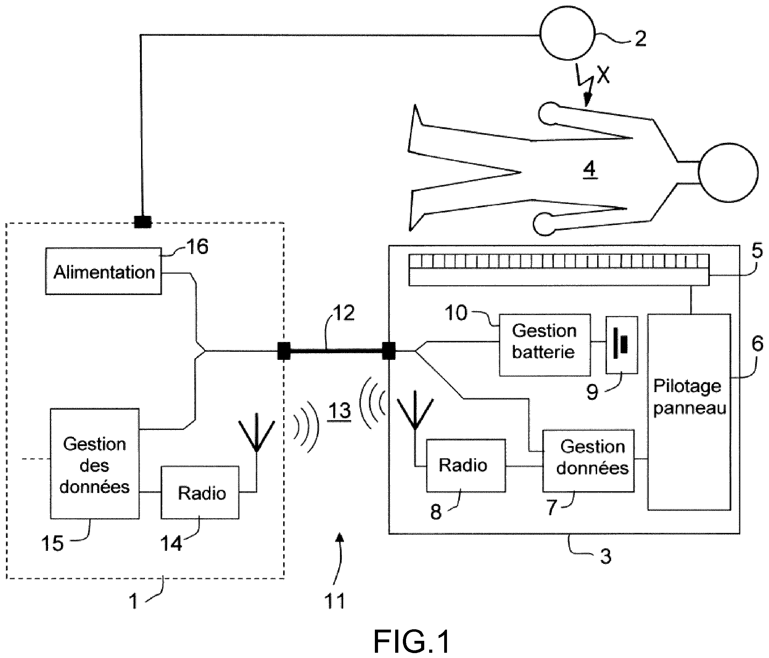

La figure 1 représente un système radiologique destiné à une utilisation médicale. Le système comporte une station de base fixe 1 un générateur de rayonnement X 2 et un détecteur de rayonnement sous forme d'une cassette portable 3. La cassette permet d'obtenir une image d'un patient 4 traversé par le rayonnement X issu du générateur 2. La cassette 3 comporte un détecteur numérique réalisé sous forme d'un panneau plat 5 relié à un module de pilotage 6 permettant de lire l'image obtenue par le panneau plat 5 et de la numériser au travers d'un convertisseur analogique numérique. La cassette mobile 3 comporte également un module de gestion de données 7, un module radio 8, une batterie 9 et un module de gestion de la batterie 10.

La station de base comporte un module radio 14, un module de gestion de données 15 et une alimentation 16.

Des moyens de communication 11 entre la cassette 3 et la station de base 1 permettent de transférer des données telles que l'image entre la cassette 3 et la station de base 1. Les données peuvent circuler soit de la station de base 1 vers la cassette 3, soit de la cassette 3 vers la station de base 1. Vers la cassette 3, il s'agit par exemple d'informations de commande du panneau plat 5 et vers la station de base 1, les données comportent par exemple des images réalisées par le panneau plat 5.

Les moyens de communication peuvent comprendre une liaison filaire amovible 12 et/ou une liaison sans fil 13. Les deux liaisons 12 et 13 sont susceptibles toutes deux de transférer les données. Les deux modules radio 8 et 14 permettent d'échanger les données entre la station de base 1 et la cassette 3. Le module de gestion de données 7 de la cassette 3 permet d'aiguiller les données reçues ou en provenance du module de pilotage 6 vers l'une des liaisons 12 ou 13. De même, dans la station de base 1, le module de gestion de données 15 permet d'aiguiller les données reçues ou en provenance d'une des liaisons 12 ou 13. L'alimentation 16 fournit l'énergie électrique nécessaire au fonctionnement des différents modules de la station de base 1 ainsi que de la cassette 3.

L'alimentation de la cassette 3 se fait par l'intermédiaire de la liaison filaire 12 ou de la batterie 9. Avantageusement, le système comporte des moyens pour recharger la batterie 9. Plus précisément, le module de gestion de la batterie 10 mesure la charge de la batterie 9 et provoque sa recharge en cas de besoin.

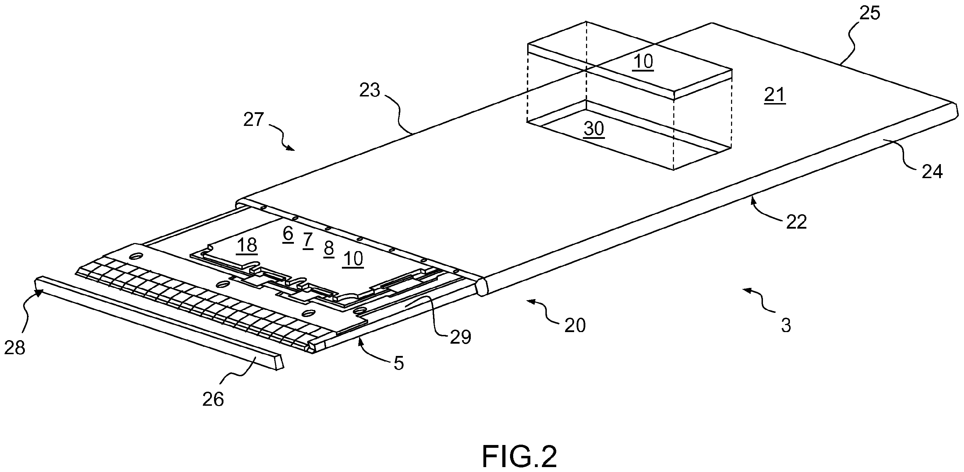

La figure 2 représente une première variante de cassette portable en vue éclatée. La cassette porte ici le repère 17 et comprend un boitier 20 ayant une forme essentiellement parallélépipédique dans lequel sont disposés le détecteur numérique 5, et une carte électronique 18 assurant la gestion du détecteur 5. Pour se référer à la figure 1, la carte électronique 18 comprend par exemple le module de pilotage 6, le module de gestion de données 7, le module radio 8, et le module de gestion de la batterie 10. Ces quatre modules ne sont donnés qu'à titre d'exemple. Ils ne sont pas obligatoires pour la mise en oeuvre de l'invention. La batterie 9 est disposée à l'extérieur du boitier 20 afin de faciliter un éventuel remplacement.

Le boitier 20 possède six faces principales 21 à 26 délimitant la forme parallélépipédique. Les six faces sont parallèles deux à deux. Le détecteur 5, sous forme d'un panneau plat, possède une surface de détection de rayonnement proche de celle des deux plus grandes faces 21 et 22. Les faces parallèles 25 et 26 sont les deux plus petites faces du boitier 20.

Le boitier 20 comprend par exemple une enveloppe 27 réalisée dans une pièce mécanique monobloc formant les cinq faces 21 à 25 de la forme essentiellement parallélépipédique dont les deux plus grandes faces 21 et 22. Le boitier 20 comprend en outre un bouchon 28 permettant d'obturer la face 26 de la forme essentiellement parallélépipédique. Alternativement, le bouchon 28 peut obturer la face 23 ou la face 24. Il est cependant avantageux de placer le bouchon 28 sur une des deux plus petites faces, dans l'exemple représenté : la face 26 afin de limiter au maximum la surface de contact entre les deux éléments 27 et 28 formant le boitier 20 afin de faciliter la réalisation de l'étanchéité du boitier 20 et d'augmenter la rigidité mécanique du boitier 20.

Le fait de réaliser une pièce monobloc sur cinq faces permet de rigidifier fortement le boitier. Plus particulièrement, les trois plus petites faces 23, 24 et 25 ceinturent le boitier 20 selon deux directions perpendiculaires, ce qui augmente la rigidité du boitier 20 en torsion autour d'axes parallèles aux deux plus grandes faces 21 et 22.

Les différents éléments disposés à l'intérieur du boitier 20 sont solidaires les uns des autres et sont glissés dans l'enveloppe 27 par la face 26 dans un mouvement de translation perpendiculairement à cette face. Ces différents éléments sont par exemple montés sur une embase 29 plate ayant une surface proche de celle des deux plus grandes faces 21 et 22. Le détecteur 5 est fixé sur l'embase 29 d'un coté de celle-ci et la carte électronique 18 est fixée sur l'embase 29 de l'autre coté de celle-ci.

La batterie 10 est logée à l'intérieur du volume parallélépipédique formée par le boitier 20. La batterie 10 est logée par l'extérieur de la casette 17 dans un embrèvement 30 réalisée dans la face 21. La face 22, opposée à la face 21, est destinée à être traversée par le rayonnement ionisant à détecter. Le détecteur numérique 5 est disposé à l'intérieur du boitier 20 du coté de la face 22.

Par le passé, la radiologie médicale utilisait des films argentiques qui étaient manipulés dans des cassettes. La norme ISO 4090 a défini les dimensions des cassettes enveloppant les films argentiques. L'épaisseur des cassettes, définie par la norme, est comprise entre 13 et 16 mm. Avantageusement, La cassette 17 répond, quant à ses dimensions, aux exigences de la norme ISO 4090. Plus particulièrement, l'épaisseur hors tout de la cassette 3 mesurée entre les deux plus grandes faces 21 et 22 est inférieure à 16 mm. Ceci permet d'utiliser les moyens de rangements de cassettes argentiques pour une cassette numérique 17.

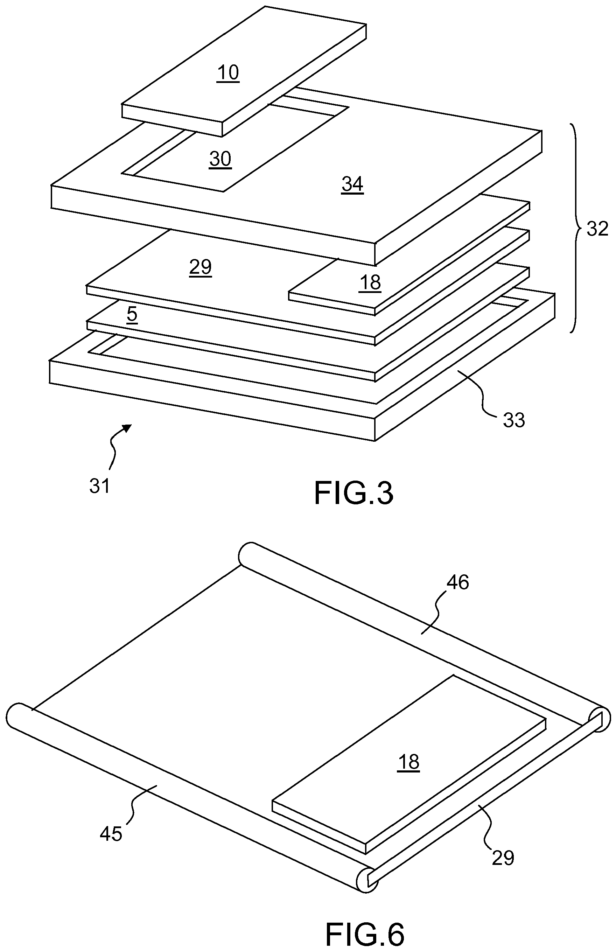

La figure 3 représente en vue éclatée une seconde variante de cassette portable, portant ici le repère 31. On retrouve, assemblé de la même façon, l'embase 29, le détecteur 5 et la carte électronique 18. On retrouve également la batterie 10. A la différence de la figure 2, le boitier diffère, il porte ici le repère 32. Il est formé de deux demi coques 33 et 34 formant chacune une des plus grandes faces du boitier 31. L'embase 29 et les différents éléments qui y sont fixés sont disposés entre les deux demi coques 33 et 34 qui sont ensuite fixées l'une à l'autre, par exemple par vissage, au niveau de la périphérie des deux grandes faces. La batterie 10 est, comme dans la première variante, logée dans un embrèvement 30 réalisé dans la demi-coque 34.

La mise en oeuvre d'une pièce monobloc pour réaliser l'enveloppe 27, comme représenté sur la figure 2 permet d'augmenter sa rigidité mécanique. En effet, dans la variante de réalisation de la figure 3, la présence d'un plan d'assemblage de deux pièces mécaniques distinctes 33 et 34 au niveau d'une des grandes faces 21 ou 22 réduit la rigidité en torsion et en flexion du boitier. Dans la variante de la figure 3, le plan d'assemblage est reporté sur une des petites faces du boitier, à savoir la face 26 dans l'exemple représenté.

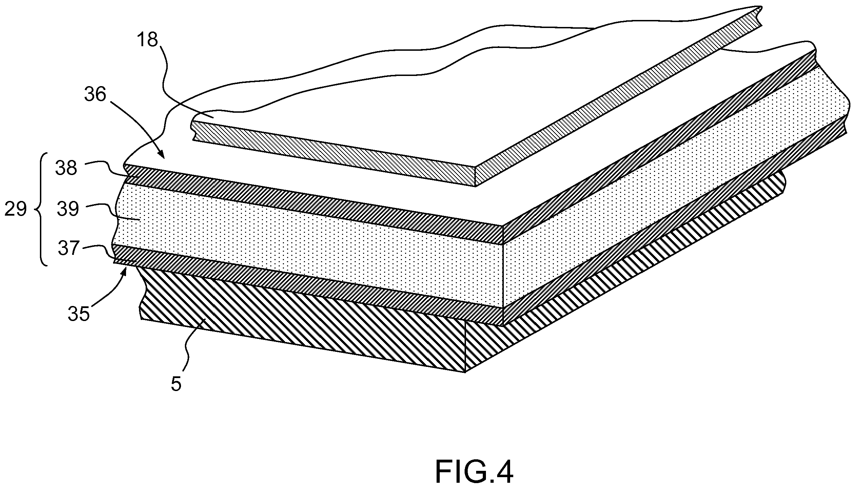

La figure 4 représente plus en détail les éléments disposés à l'intérieur du boitier 20 ou du boitier 32, à savoir l'embase 29 sur laquelle sont fixés le détecteur 5 et la carte électronique 18. L'embase 29 a la forme d'une plaque globalement plane et comprend deux faces principales 35 et 36 opposées l'une à l'autre. Le détecteur 5 est fixé sur la face 35 et la carte électronique 18 sur la face 36. L'ensemble formé par l'embase 29, la carte électronique 18 et le détecteur 5 est autoporté. On peut manipuler cet ensemble avant son assemblage dans l'un des boitiers 20 ou 32. Dans une cassette radiologique parallélépipédique comme représenté dans les deux variantes des figures 2 et 3, on cherche à utiliser au mieux la surface des plus grandes faces du parallélépipède pour la détection du rayonnement X. La surface du détecteur 5 est pratiquement égale à celle des deux grandes faces 21 et 22. L'embase 29 assure la fonction de support mécanique du détecteur 5 qui occupe la quasi totalité de la surface de l'embase 29.

L'embase est monobloc. Elle constitue une pièce mécanique autonome. L'embase 29 est formée d'un empilement comprenant au moins deux peaux 37 et 38 externes formant les deux faces principales de l'embase 29, respectivement 35 et 36, et un matériau interne 39, par exemple formé d'un matériau alvéolaire disposé entre les deux peaux 37 et 38. Un matériau alvéolaire, du fait de sa structure, possède une résistance thermique élevée. Comme matériau alvéolaire, il est possible de mettre en oeuvre une mousse, un bois léger comme du balsa ou une structure en nid d'abeille. En revanche, les peaux 37 et 38 sont avantageusement réalisées dans un matériau plein. La densité des peaux est avantageusement plus forte que celle su matériau interne. La résistance mécanique des deux peaux externes 37 et 38 est avantageusement supérieure à la résistance mécanique de la mousse 39. La mousse 39 et les deux peaux 37 et 38 sont globalement planes et ont une surface sensiblement égale à celle du détecteur 5.

Il est possible de concevoir une embase à partir d'empilements comprenant un plus grand nombre de constituants pour former par exemple une alternance de couches de peau et de mousse.

Lorsqu'on cherche à réaliser une cassette rentrant dans les dimensions imposées par la norme ISO 4090, l'épaisseur hors tout de la cassette ne doit pas dépasser 16 mm. Dans cette épaisseur, on doit disposer le boitier, le détecteur 5, l'embase 29 et la carte électronique 18 équipée de ses composants. Lors de la conception de la cassette, on est contraint par les éléments actifs de la cassette, le détecteur 5 et la carte électronique 18, ce qui laisse peu d'épaisseur disponible pour les pièces mécaniques et notamment pour l'embase 29. Des essais internes réalisés par la demanderesse ont montré que l'épaisseur de l'embase 29 ne devait pas dépasser 2 mm. On peut admettre une épaisseur jusqu'à 3 mm mais cela entraine des contraintes plus importantes sur les différents éléments entrant dans l'empilement de 16 mm. A ce jour aucun empilement mettant en oeuvre une mousse montée en sandwich entre deux peaux n'a été mis en oeuvre pour des épaisseurs si fines. Pour réaliser un support mécanique d'une telle épaisseur, l'idée largement répandue consiste à réaliser une embase dans un matériau homogène. On profite ainsi de la raideur du matériau retenu dans toute son épaisseur.

Néanmoins, l'amélioration de la résistance mécanique de l'embase se fait au détriment de sa tenue aux contraintes thermiques d'une cassette radiologique. Plus précisément, les matériaux ayant une bonne résistance mécanique ont le pus souvent une bonne conductivité thermique. La présence de la carte électronique 18 en regard d'une partie du détecteur 5 perturbe alors localement le fonctionnement du détecteur 5.

Plus précisément, la réalisation de l'embase 29 dans un matériau métallique homogène comme par exemple l'aluminium ou le titane est bien indiquée pour une bonne résistance mécanique. En revanche ces métaux transmettent particulièrement bien la chaleur et génèrent des artéfacts difficiles à corriger. On peut améliorer la situation thermique en réalisant l'embase 29 en matériau composite, comme par exemple des fibres noyées dans de la résine. La faible épaisseur de l'embase 29 ne permet pas d'améliorer complètement la situation thermique.

La présence de la mousse 39 dans l'empilement de l'embase 29 permet de réaliser une rupture de pont thermique dans une direction transversale de l'embase. Les peaux 37 et 38 possèdent une conductivité thermique supérieure à la conductivité thermique de la mousse 39. La carte électronique 18 diffuse la chaleur émise lors de son fonctionnement vers la peau 38 sur laquelle elle est fixée. Du fait de la différence de conductivité thermique surfacique et transversale de l'embase 29, la chaleur émise par la carte électronique 18 tend à diffuser plus facilement par conduction sur toute la surface de la peau 38 que transversalement vers l'autre peau 37. On obtient ainsi une température plus homogène de la peau 37 en contact avec le détecteur 5, qu'en l'absence de rupture de pont thermique.

Il est bien entendu possible de réaliser d'autres empilements hétérogènes pour réaliser l'embase 29, notamment des empilements comprenant plus de trois couches distinctes.

Pour revenir à l'empilement décrit, avantageusement, les deux peaux 37 et 38 sont réalisées dans un même matériau afin de simplifier la réalisation de l'embase 29. Les deux peaux 37 et 38 peuvent être réalisées dans un alliage métallique, tel que par exemple un alliage à base de fer, de cuivre ou d'aluminium. Bien que certains alliages de fer tel que des aciers inoxydables à structure austénitique ait des conductivités thermiques relativement faibles, de l'ordre de 15 W.m⁻¹.K⁻¹ voire de 10 W.m⁻¹.K⁻¹, il est possible d'utiliser ces alliages pour leur propriété de résistance mécanique. On rappelle que le cuivre a une conductivité thermique de 390 W.m⁻¹.K⁻¹ et que l'aluminium de 237 W.m⁻¹.K⁻¹.

Il est également possible de former les peaux 37 et 38 dans un matériau composite comprenant des fibres et de la résine noyant les fibres. On peut mettre en oeuvre de nombreuses fibres telles que par exemple des fibres de verre ou de carbone. Les fibres de carbone présentent l'avantage d'être conductrices de l'électricité. Elles peuvent ainsi remplir une fonction de blindage électromagnétique entre la carte électronique 18 et le détecteur 5. Pour améliorer la tenue mécanique, on peut également noyer des fibres d'aramide. Il est bien entendu possible de mélanger différent types de fibres en fonction du résultat recherché.

Afin d'améliorer l'homogénéité de la température de la peau 38, celle-ci peut comprendre des fibres dont la conductivité thermique est supérieure à supérieure à 30 W.m⁻¹.K⁻¹. A titre d'exemple, il existe des fibres de carbone à base de brai, connu dans la littérature anglo-saxonne sous le nom de : « pitch based carbon fiber » dont la conductivité thermique dépasse 500 W.m⁻¹.K⁻¹. La peau 37 peut bien entendu comprendre le même type de fibres.

Pour réaliser les peaux 37 et 38, on peut utiliser des fibres organisées en tissus ou en mat qui peuvent être secs ou pré imprégnés de résine.

Plus précisément, les fibres de carbone peuvent être disposées dans le moule sous forme de fibres pré imprégnées en plusieurs couches se recouvrant II est également possible de disposer des fibres sèches dans le moule puis d'injecter de la résine selon une technologie connue sous le nom de moulage par transfert de résine ou « Resin Transfer Modling » dans la littérature anglo-saxonne. Ce procédé de fabrication permet également d'améliorer l'état de surface de faces 35 et 36. L'état de surface permet de faciliter la mise en place de la carte électronique 18 et du détecteur 5, notamment en garantissant une épaisseur constante de l'embase 29.

Alternativement aux fibres organisées en tissus ou en mat, on peut aussi utiliser des fibres unidirectionnelles. Dans ce cas, afin d'assurer une bonne résistance mécanique, les fibres unidirectionnelles sont disposées en plusieurs couches croisées. Une disposition particulière des fibres unidirectionnelles présente un avantage au niveau de la conductivité thermique.

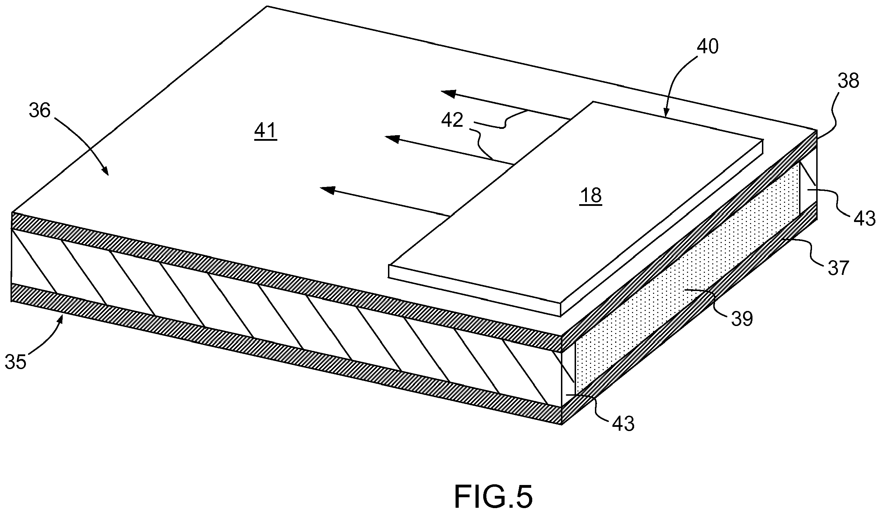

La figure 5 permet de mieux préciser cet avantage. Plus précisément, il est courant que la carte électronique 18 recouvre une première partie 40 seulement de l'embase 29. Une seconde partie 41 de l'embase 29 n'est pas recouverte par la carte électronique 18. Pour la couche de fibres unidirectionnelles disposée au plus près de la carte électronique 18, il est avantageux de diriger les fibres qu'elle contient de la première partie 40 vers la seconde partie 41. Cette direction est représentée par des flèches 42 sur la figure 5. Cette orientation des fibres est réalisée de façon à conduire de la chaleur générée par la carte électronique18 lors de son fonctionnement préférentiellement de la partie 40 vers la partie 41. Cela permet une meilleure homogénéité de la température de la peau 38 sur toute sa surface et plus généralement sur toute la surface de l'embase 29.

Pour obtenir une embase de 2 mm d'épaisseur, on peut par exemple mettre en oeuvre une mousse 39 d'épaisseur 1,2 mm et deux peaux en matériaux composites à base de fibres de carbone de 0,4 mm d'épaisseur chacune. Un exemple de mousse pouvant être mise en oeuvre est une mousse de Poly méthacrylate de méthyle, connue par son abréviation anglo-saxonne PMMA pour Poly Methyl MethAcrylate. Ce type de mousse a une conductivité thermique de l'ordre de 0,04 W.m⁻¹.K⁻¹.

De façon plus générale, il est avantageux de choisir les matériaux des peaux et de la mousse de telle sorte que le rapport entre la conductivité thermique surfacique de l'embase et la conductivité thermique transversale de l'embase est supérieur à 100.

Afin d'améliorer la tenue mécanique de l'embase 29, celle-ci peut comprendre en périphérie de ses faces principales 35 et 36 un renfort mécanique 43 reliant les peaux 37 et 38. Le renfort 43 est avantageusement réalisé dans le matériau mis en oeuvre pour les peaux 37 et 38. Le renfort 43 peut suivre en totalité le périmètre des faces 35 et 36. Le renfort 43 peut alternativement ne suivre qu'une partie du périmètre des faces 35 et 36 comme représenté sur la figure 5.

Le renfort 43 est utilisé pour améliorer l'homogénéité de la température des deux peaux 37 et 38. A cet effet, le renfort 43 possède une conductivité thermique supérieure à la conductivité thermique transversale de l'embase 29 définie en l'absence du renfort 43. Le renfort 43 réalise ainsi un pont thermique périphérique entre les deux faces de l'embase 29. En effet les principales sources de chaleur de la cassette sont des composants électroniques situés sur la carte électronique 18. Lorsque ces composants dissipateurs sont placés loin de la périphérie de la carte électronique 18, la chaleur qu'ils émettent se dissipe sur la peau 38 avant d'atteindre le renfort 43 dont la température est de ce fait sensiblement homogène. Le pont thermique réalisé par le renfort 43 permet alors de conserver une bonne homogénéité de la température de la peau 37 sur laquelle est fixé le détecteur numérique 5.

Lorsque les peaux 37 et 38 sont réalisées en matériau composites, du fait de la présence de fibres disposées dans le plan de la peau, leur conductivité thermique surfacique et meilleure que leur conductivité thermique transversale. Il est alors difficile de réaliser le renfort 43 à l'aide d'un tel matériau pour remplir la fonction de pont thermique entre les deux peaux 37 et 38. Pour ce faire, il faudrait intégrer dans le renfort 43 des fibres transversales. Alternativement, pour simplifier la réalisation du renfort 43, on peut le réaliser dans un matériau homogène tel que par exemple un alliage d'aluminium qui assure à la fois la fonction de renfort mécanique et de pont thermique entre les deux peaux 37 et 38.

Dans le cas de la première variante représentée sur la figure 2, l'embase 29, le détecteur 5 et la carte électronique 18 sont disposés dans l'enveloppe 27 par la face 26. Plus précisément, l'ensemble formé par ces trois éléments assemblés sont glissés dans l'enveloppe 27 par la face 26. Le bouchon 28 obture ensuite la face 26. Des bords de l'embase 29 assurent le guidage de l'ensemble lors de l'insertion dans l'enveloppe 27. Pour faciliter cette insertion, on peut rapporter sur l'embase 29 deux profilés 45 et 46 assurant la fonction de glissière qui permettent de faciliter la mise en position de l'embase dans le boitier 20. Ces profilés apparaissent sur la figure 6. Afin de limiter la chaîne de cote entre l'enveloppe 27 et l'embase 29, on peut fixer les profilés 45 et 46 sur l'embase 29, par exemple par collage puis usiner les deux profilés conjointement de façon à obtenir une bonne précision dans la distance séparant les deux profilés 45 et 46.

## Revendications

1. Cassette radiologique portable comprenant un détecteur numérique de rayonnement ionisant (5) sous forme d'un panneau plat, une carte électronique (18) assurant la gestion du détecteur numérique (5) et un boitier (20 ; 32) assurant la protection mécanique du détecteur (5) et de la carte électronique (18), une embase (29) monobloc plane supportant le détecteur (5) sur une première de ses faces principales (35) et la carte électronique (18) sur une seconde de ses faces principales (36), les deux faces principales (35, 36) étant opposées, l'embase (29) étant formée d'un empilement hétérogène réalisé de façon à ce que la conductivité thermique surfacique de l'embase (29) soit supérieure à la conductivité thermique transversale de l'embase (29), l'embase (29) comprenant deux peaux externes (37, 38) formant les deux faces principales (35, 36) et un matériau interne (39) disposé entre les deux peaux externes (37, 38), le matériau interne (39) et les deux peaux externes (37, 38) ayant une surface sensiblement égale à celle du détecteur (5), la conductivité thermique des peaux externes (37, 38) étant supérieure à la conductivité thermique du matériau interne, **caractérisée en ce que** l'embase (29) comprend en périphérie de ses faces principales (35, 36) un renfort mécanique (43) reliant les peaux (37, 38) et **en ce que** le renfort (43) possède une conductivité thermique supérieure à la conductivité thermique transversale de l'embase (29) définie en l'absence du renfort (43).

2. Cassette radiologique selon la revendication 1, **caractérisée en ce qu'**un rapport entre la conductivité thermique surfacique de l'embase et la conductivité thermique transversale de l'embase est supérieur à 100.

3. Cassette radiologique selon l'une des revendications précédentes, **caractérisée en ce que** le boitier (20 ; 32) a une forme essentiellement parallélépipédique dont les deux plus grandes faces (21, 22) sont parallèles au détecteur (5), et **en ce que** l'épaisseur hors tout de la cassette (3) mesurée entre les deux plus grandes faces (21, 22) est inférieure à 16 mm.

4. Cassette radiologique selon l'une des revendications précédentes, **caractérisée en ce que** le matériau interne est formé d'un matériau alvéolaire (39).

5. Cassette radiologique selon l'une quelconque des revendications précédentes, **caractérisée en ce que** les deux peaux externes (37, 38) sont formées dans un matériau composite comprenant des fibres et de la résine noyant les fibres.

6. Cassette radiologique selon la revendication 5, **caractérisée en ce que** la peau (38) formant la seconde de face principale (36) sur laquelle est fixée la carte électronique (18) comprend des fibres dont la conductivité thermique est supérieure à 30 W.m⁻¹.K⁻¹.

7. Cassette radiologique selon l'une quelconque des revendications 5 ou 6, **caractérisée en ce que** la carte électronique (18) recouvre une première partie (40) de l'embase (29) et **en ce que** la peau (38) formant la seconde de face principale (36) sur laquelle est fixée la carte électronique (18) comprend plusieurs couches de fibres unidirectionnelles, et **en ce que** pour la couche disposée au plus près de la carte électronique (18), les fibres unidirectionnelles sont dirigées (42) de la première partie (40) vers une seconde partie (41) de l'embase (29) non recouverte par la carte électronique (18) de façon à conduire de la chaleur générée par la carte électronique (18) lors de son fonctionnement, préférentiellement de la première partie (40) vers la seconde partie (41) de l'embase (29).

8. Cassette radiologique selon l'une quelconque des revendications précédentes, **caractérisée en ce que** la conductivité thermique de la peau (38) formant la seconde de face principale (36) sur laquelle est fixée la carte électronique (18) est supérieure à 10 W.m⁻¹.K⁻¹.

9. Cassette radiologique selon l'une des revendications précédentes, **caractérisée en ce que** le boitier (20) a une forme essentiellement parallélépipédique dont les deux plus grandes faces (21, 22) sont parallèles au détecteur (5), **en ce qu'**une première (22) des deux grandes faces est destinée à être traversée par le rayonnement ionisant, **en ce que** le boitier (20), comprend une enveloppe (27) réalisée dans une pièce mécanique monobloc formant cinq premières faces (21, 22, 23, 24, 25) de la forme essentiellement parallélépipédique dont les deux plus grandes faces (21, 22), et un bouchon (28) permettant d'obturer une sixième face (26) de la forme essentiellement parallélépipédique et **en ce qu'**un ensemble formé par l'embase (29), le détecteur (5) et la carte électronique (18) sont insérés dans l'enveloppe (27) par la sixième face (26).

10. Cassette radiologique selon la revendication 9, **caractérisée en ce que** l'embase (29) comprend deux profilés (45, 46) rapportés assurant la fonction de glissière lors de l'insertion de l'ensemble formé par l'embase (29), le détecteur (5) et la carte électronique (18) dans l'enveloppe (27).

11. Cassette radiologique selon l'une des revendications précédentes, **caractérisée en ce que** la résistance mécanique des deux peaux externes (37, 38) est supérieure à la résistance mécanique du matériau interne (39).

12. Cassette radiologique selon l'une des revendications précédentes, **caractérisée en ce qu'**une épaisseur de l'embase est inférieure à 3 mm.

## Patentansprüche

1. Tragbare radiologische Kassette, die Folgendes umfasst: einen digitalen Ionisierungsstrahlungsdetektor (5) in Form einer flachen Platte, eine elektronische Karte (18), die die Verwaltung des digitalen Detektors (5) gewährleistet, und ein Gehäuse (20; 32), das den mechanischen Schutz des Detektors (5) und der elektronischen Karte (18) gewährleistet, wobei ein ebener einteiliger Sitz (29) den Detektor (5) auf einer ersten seiner Hauptflächen (35) und die elektronische Karte (18) auf einer zweiten seiner Hauptflächen (36) trägt, wobei die beiden Hauptflächen (35, 36) einander gegenüberliegen, wobei der Sitz (29) von einem heterogenen Stapel gebildet wird, so realisiert, dass die Wärmeleitfähigkeit der Oberfläche des Sitzes (29) höher ist als die transversale Wärmeleitfähigkeit des Sitzes (29), wobei der Sitz (29) zwei Außenhäute (37, 38), die die beiden Hauptflächen (35, 36) bilden, und ein Innenmaterial (39) aufweist, das zwischen den beiden Außenhäuten (37, 38) angeordnet ist, wobei das Innenmaterial (39) und die beiden Außenhäute (37, 38) eine Oberfläche im Wesentlichen gleich der des Detektors (5) haben, wobei die Wärmeleitfähigkeit der Außenhäute (37, 38) höher ist als die Wärmeleitfähigkeit des Innenmaterials, **dadurch gekennzeichnet, dass** der Sitz (29) an der Peripherie seiner Hauptflächen (35, 36) eine die Häute (37, 38) verbindende mechanische Verstärkung (43) aufweist, und dadurch, dass die Verstärkung (43) eine Wärmeleitfähigkeit hat, die höher ist als die in Abwesenheit der Verstärkung (43) definierte transversale Wärmeleitfähigkeit des Sitzes (29).

2. Radiologische Kassette nach Anspruch 1, **dadurch gekennzeichnet, dass** ein Verhältnis zwischen der Wärmeleitfähigkeit der Oberfläche des Sitzes und der transversalen Wärmeleitfähigkeit des Sitzes größer als 100 ist.

3. Radiologische Kassette nach einem der vorherigen Ansprüche, **dadurch gekennzeichnet, dass** das Gehäuse (20; 32) im Wesentlichen die Form eines Quaders hat, dessen zwei größten Flächen (21, 22) parallel zum Detektor (5) sind, und dadurch, dass die Dicke außerhalb der Kassette (3), gemessen zwischen den zwei größten Flächen (21, 22), kleiner als 16 mm ist.

4. Radiologische Kassette nach einem der vorherigen Ansprüche, **dadurch gekennzeichnet, dass** das Innenmaterial die Form eines Zellmaterials (39) hat.

5. Radiologische Kassette nach einem der vorherigen Ansprüche, **dadurch gekennzeichnet, dass** die beiden Außenhäute (37, 38) aus einem Verbundmaterial gebildet sind, das Fasern und die Fasern einbettendes Harz umfasst.

6. Radiologische Kassette nach Anspruch 5, **dadurch gekennzeichnet, dass** die Haut (38), die die zweite Hauptfläche (36) bildet, auf der die elektronische Karte (18) befestigt ist, Fasern umfasst, deren Wärmeleitfähigkeit höher als 30 W.m⁻¹.K⁻¹ ist.

7. Radiologische Kassette nach einem der Ansprüche 5 oder 6, **dadurch gekennzeichnet, dass** die elektronische Karte (18) einen ersten Teil (40) des Sitzes (29) bedeckt, und dadurch, dass die Haut (38), die die zweite Hauptfläche (36) bildet, auf der die elektronische Karte (18) befestigt ist, mehrere unidirektionale Faserschichten umfasst, und dadurch, dass für die am nächsten an der elektronischen Karte (18) angeordnete Schicht die unidirektionalen Fasern vom ersten Teil (40) zu einem zweiten Teil (41) des Sitzes (29) geleitet (42) werden, der nicht von der elektronischen Karte (18) bedeckt wird, um die von der elektronischen Karte (18) bei deren Betrieb erzeugte Wärme vorzugsweise vom ersten Teil (40) zum zweiten Teil (41) des Sitzes (29) abzuführen.

8. Radiologische Kassette nach einem der vorherigen Ansprüche, **dadurch gekennzeichnet, dass** die Wärmeleitfähigkeit der Haut (38), der die zweite Hauptfläche (36) bildet, auf der die elektronische Karte (18) befestigt ist, größer als 10 W.m⁻¹.K⁻¹ ist.

9. Radiologische Kassette nach einem der vorherigen Ansprüche, **dadurch gekennzeichnet, dass** das Gehäuse (20) im Wesentlichen die Form eines Quaders hat, dessen zwei größten Flächen (21, 22) parallel zum Detektor (5) sind, und dadurch, dass eine erste (22) der zwei größten Flächen dazu bestimmt ist, von der Ionisierungsstrahlung durchquert zu werden, dadurch, dass das Gehäuse (20) eine Umhüllung (27) aufweist, realisiert aus einem mechanischen Einzelblock, der fünf erste Flächen (21, 22, 23, 24, 25) im Wesentlichen mit der Form eines Quaders bildet, dessen zwei größten Flächen (21, 22) und ein Pfropfen (28) das Verdecken einer sechsten Fläche (26) im Wesentlichen mit der Form des Quaders zulässt, und dadurch, dass eine durch den Sitz (29), den Detektor (5) und die elektronische Karte (18) gebildete Baugruppe durch die sechste Fläche (26) in die Umhüllung (27) eingeführt wird.

10. Radiologische Kassette nach Anspruch 9, **dadurch gekennzeichnet, dass** der Sitz (29) zwei angesetzte Profile (45, 46) umfasst, die die Gleitfunktion beim Einführen der durch den Sitz (29), den Detektor (5) und die elektronische Karte (18) gebildeten Baugruppe in die Umhüllung (27) gewährleistet.

11. Radiologische Kassette nach einem der vorherigen Ansprüche, **dadurch gekennzeichnet, dass** der mechanische Widerstand der beiden Außenhäute (37, 38) höher ist als der mechanische Widerstand des Innenmaterials (39).

12. Radiologische Kassette nach einem der vorherigen Ansprüche, **dadurch gekennzeichnet, dass** eine Dicke des Sitzes kleiner als 3 mm ist.

## Claims

1. A portable radiological cassette comprising a digital ionizing radiation detector (5) in the form of a flat panel, an electronic board (18) ensuring the control of the digital detector (5), and a casing (20; 32) ensuring the mechanical protection of the detector (5) and of the electronic board (18), a flat seating (29) in a single piece supporting the detector (5) on a first one of its main faces (35) and the electronic board (18) on a second one of its main faces (36), the two main faces (35, 36) being opposite, the seating (29) being formed by a heterogeneous stack produced such that the surface thermal conductivity of the seating (29) is greater than the transverse thermal conductivity of the seating (29), the seating (29) comprising two outer skins (37, 38) which form the two main faces (35, 36), and an inner material (39) which is arranged between the two outer skins (37, 38), the inner material (39) and the two outer skins (37, 38) having a surface area which is substantially equal to that of the detector (5), the thermal conductivity of the outer skins (37, 38) being greater than the thermal conductivity of the inner material, **characterised in that** the seating (29) comprises, on the periphery of its main faces (35, 36), a mechanical reinforcement (43) which connects the skins (37, 38), and **in that** the reinforcement (43) has a thermal conductivity which is greater than the transverse thermal conductivity of the seating (29) defined in the absence of the reinforcement (43).

2. The radiological cassette as claimed in claim 1, **characterised in that** a ratio between the surface thermal conductivity of the seating and the transverse thermal conductivity of the seating is greater than 100.

3. The radiological cassette as claimed in one of the preceding claims, **characterised in that** the casing (20; 32) has a substantially parallelepipedal form, the two largest faces (21, 22) of which are parallel to the detector (5), and **in that** the overall thickness of the cassette (3) measured between the two largest faces (21, 22) is smaller than 16 mm.

4. The radiological cassette as claimed in one of the preceding claims, **characterised in that** the inner material is formed by an alveolar material (39).

5. The radiological cassette as claimed in one of the preceding claims, **characterised in that** the two outer skins (37, 38) are formed from a composite material comprising fibres and resin in which the fibres are embedded.

6. The radiological cassette as claimed in claim 5, **characterised in that** the skin (38) which forms the second main face (36) on which the electronic board (18) is secured comprises fibres, the thermal conductivity of which is greater than 30 W.m⁻¹.K⁻¹.

7. The radiological cassette as claimed in either of claims 5 or 6, **characterised in that** the electronic board (18) covers a first part (40) of the seating (29) and **in that** the skin (38) which forms the second main face (36) on which the electronic board (18) is secured comprises a plurality of layers of one-way fibres, and **in that**, for the layer which is arranged closest to the electronic board (18), the one-way fibres are directed (42) from the first part (40) towards a second part (41) of the seating (29) which is not covered by the electronic board (18), such as to conduct heat which is generated by the electronic board (18) during its operation, preferably from the first part (40) to the second part (41) of the seating (29).

8. The radiological cassette as claimed in any one of the preceding claims, **characterised in that** the thermal conductivity of the skin (38) which forms the second main face (36) on which the electronic board (18) is secured is greater than 10 W.m⁻¹.K⁻¹.

9. The radiological cassette as claimed in one of the preceding claims, **characterised in that** the casing (20) has a substantially parallelepipedal form, the two largest faces (21, 22) of which are parallel to the detector (5), **in that** a first one (22) of the two large faces is designed to have the ionizing radiation passing through it, **in that** the casing (20) comprises an envelope (27) produced in the form of a mechanical part in a single piece which forms five first faces (21, 22, 23, 24, 25) of the substantially parallelepipedal form, the two largest faces (21, 22) of which and a stopper (28) make it possible to shut a sixth face (26) of the substantially parallelepipedal form, and **in that** an assembly formed by the seating (29), the detector (5) and the electronic board (18) is inserted into the envelope (27) by the sixth face (26).

10. The radiological cassette as claimed in claim 9, **characterised in that** the seating (29) comprises two added-on profiles (45, 46) which ensure the function of a slide during the insertion of the assembly formed by the seating (29), the detector (5) and the electronic board (18) into the envelope (27).

11. The radiological cassette as claimed in one of the preceding claims, **characterised in that** the mechanical resistance of the two outer skins (37, 38) is greater than the mechanical resistance of the inner material (39).

12. The radiological cassette as claimed in one of the preceding claims, **characterised in that** a thickness of the seating is less than 3 mm.
